(19) **Europäisches Patentamt**

**European Patent Office**

**Office européen des brevets**

(11) **EP 0 663 219 B1**

(12) **FASCICULE DE BREVET EUROPEEN**

(45) Date de publication et mention
de la délivrance du brevet:
**20.03.2002 Bulletin 2002/12**

(51) Int Cl.⁷: **A61N 1/37**, A61N 1/365

(21) Numéro de dépôt: **94403060.0**

(22) Date de dépôt: **30.12.1994**

(54) **Dispositif implantable actif**

Aktive implantierbare Vorrichtung

Active implantable device

(84) Etats contractants désignés:
**BE CH DE ES FR GB IT LI SE**

(30) Priorité: **31.12.1993 FR 9315960**

(43) Date de publication de la demande:
**19.07.1995 Bulletin 1995/29**

(73) Titulaire: **ELA MEDICAL**
**F-92541 Montrouge Cédex (FR)**

(72) Inventeurs:
• **Dalmolin, Renzo**
**F-92320 Chatillon (FR)**

• **Paris, Catherine**
**F-92120 Montrouge (FR)**

(74) Mandataire: **Laget, Jean-Loup**
**Cabinet Loyer,**
**78, avenue Raymond Poincaré**
**75116 Paris (FR)**

(56) Documents cités:
**EP-A- 0 011 932        EP-A- 0 151 689**
**EP-A- 0 429 025        EP-A- 0 493 222**
**EP-A- 0 555 988        EP-A- 0 576 114**
**GB-A- 2 195 538        US-A- 5 188 117**

**Description**

L'invention concerne un dispositif implantable actif, par exemple du type asservi à un paramètre physiologique tel que la ventilation-minute.

La famille des dispositifs implantables actifs comprend notamment les stimulateurs cardiaques et les défibrillateurs cardiaques.

Un dispositif implantable actif peut être perturbé par la présence d'ondes électromagnétiques en provenance de diverses sources telles que les radiotéléphones, ou les téléviseurs, par exemple.

Dans le cas d'un stimulateur à fréquence asservie, ces ondes électromagnétiques sont perçues par le capteur d'asservissement comme des variations du paramètre physiologique. Il en résulte que la fréquence de stimulation est accélérée sans raison valable.

Le document GB 2.195.538 décrit un circuit de réjection du bruit pour stimulateur cardiaque implantable. Au cours de chaque cycle cardiaque, le seuil de détection est fixé par référence au niveau de bruit du signal endocavitaire au cours d'une période réfractaire relative renouvelable. Ce circuit permet de s'affranchir des signaux parasites d'origine musculaire.

Un but de l'invention est de proposer un dispositif implantable actif qui prenne en compte le bruit constitué par les ondes électromagnétiques parasites afin de permettre au dispositif implantable actif de prendre une option de fonctionnement ou une décision, comme de suspendre un asservissement lorsque ce bruit dépasse un seuil.

L'invention a pour objet un dispositif implantable actif asservi à un paramètre physiologique, comportant une chaîne électronique de mesure d'une grandeur représentative du paramètre physiologique et un dispositif pour assurer l'asservissement d'une fonction du dispositif implantable actif, caractérisé en ce que :

- ladite grandeur est mesurée pour déterminer une valeur du bruit ;

- ladite valeur du bruit est comparée à un seuil, et

- lorsque la valeur du bruit dépasse le seuil, ce dépassement est pris en compte par ledit dispositif en vue d'une suspension éventuelle de la fonction.

Selon d'autres caratéristiques de l'invention :

- la fonction du dispositif implantable est l'asservissement à la fréquence de stimulation cardiaque ;

- le paramètre physiologique est la ventilation-minute et la grandeur représentative est la tension entre une électrode de recueil et le boîtier du stimulateur ;

- la tension est mesurée pendant une injection de courant pour déterminer l'impédance pulmonaire dynamique, puis, en l'absence d'injection de courant pour déterminer le bruit, la tension correspondant au bruit est comparée à une valeur de seuil, et lorsque la tension correspondant au bruit dépasse la valeur de seuil, ce dépassement est pris en compte par ledit dispositif en vue d'une suspension éventuelle de la fonction ;

- la tension est mesurée pendant une injection de courant pour déterminer l'impédance pulmonaire dynamique et en dehors d'une injection de courant pour déterminer le bruit, au moyen de la même chaîne électronique de mesure ;

- l'impédance pulmonaire dynamique est définie par différence entre l'impédance pulmonaire mesurée et l'impédance pulmonaire statique obtenue par intégration des mesures précédentes ;

- en cas de dépassement de la valeur de seuil un compteur est incrémenté et le dispositif suspend la fonction lorsque le compteur atteint un total prédéterminé ;

- la mesure de ladite grandeur et la détermination de la valeur du bruit sont effectuées seulement lorsque la mesure de la grandeur représentative d'un paramètre physiologique dépasse un seuil prédéterminé.

D'autres caractéristiques ressortent de la description qui suit faite avec référence au dessin annexé sur lequel on peut voir :

Figure 1 : un schéma synoptique d'une chaîne électronique de mesure de l'impédance pulmonaire dynamique dans un stimulateur cardiaque selon l'invention, du type à fréquence asservie à un paramètre physiologique qui, dans cet exemple, est la ventilation-minute ;

Figure 2 : un schéma synoptique d'une chaîne électronique de mesure du bruit selon un mode de réalisation de l'invention ;

Figure 3 : un schéma synoptique d'une chaîne électronique de mesure du bruit selon un autre mode de réalisation de l'invention.

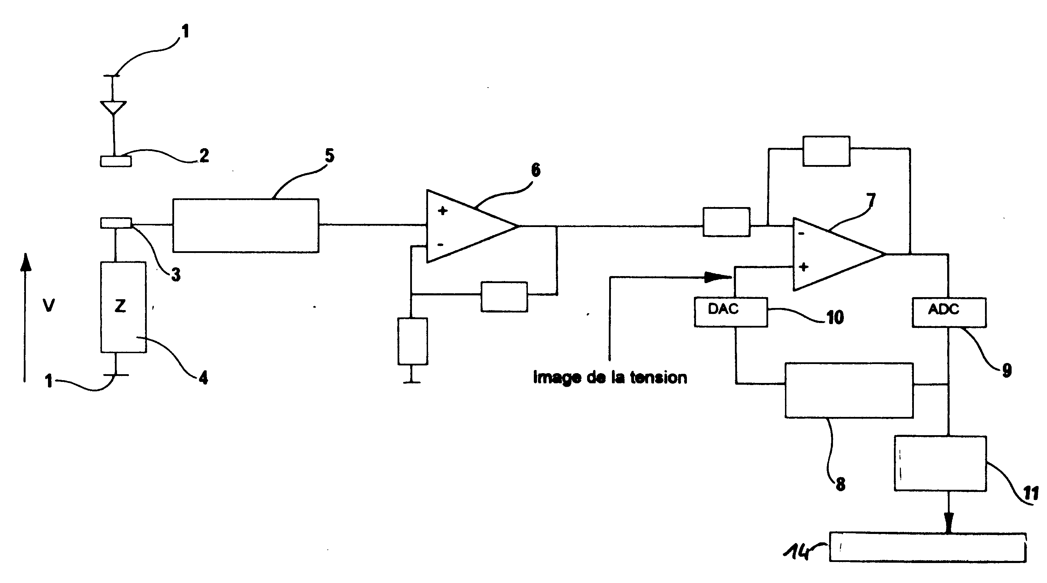

Sur la figure 1, le boîtier du stimulateur cardiaque implanté est symbolisé en 1, l'électrode d'injection, par exemple distale, en 2 et l'électrode de recueil, par exemple proximale, en 3. L'injection de courant étant assurée en 2, l'impédance pulmonaire étant symbolisée en 4 entre l'électrode de recueil 3 et le boîtier, la tension V mesurée entre le boîtier 1 et l'électrode de recueil 3 permet de déterminer la valeur Z de l'impédance pulmonaire.

Les variations du volume respiratoire se traduisent par des variations d'impédance qui sont mesurées.

La chaîne électronique de mesure comprend un filtre passe-haut 5, un amplificateur 6, un amplificateur 7 avec une boucle de contre-réaction comportant un intégrateur 8 placé entre un convertisseur analogique-numérique 9 et un convertisseur numérique-analogique 10, et enfin un filtre passe-bas 11 dont le code de sortie est lu par un microprocesseur 14.

Le fonctionnement de la chaîne s'analyse de la manière suivante : un courant est injecté sur une électrode (2) et la tension liée à l'impédance pulmonaire est mesurée entre l'autre électrode (3) et le boîtier 1. Les électrodes peuvent être localisées dans l'oreillette ou dans le ventricule.

La tension recueillie est filtrée en 5, amplifiée en 6, échantillonnée. La tension obtenue est alors diminuée de la tension correspondant à l'impédance statique, déterminée par intégration numérique des tensions obtenues au cours des cycles précédents.

La tension résultante à la sortie de l'amplificateur 7 correspond alors à l'impédance dynamique. Après conversion analogique-numérique en 9 cette tension est filtrée en 11 et le code numérique résultant est lu par le microprocesseur 14 qui réalise son algorithme d'asservissement.

L'intégration en 8 de la tension sous forme numérique issue de 9 permet la détermination de l'impédance statique sous la forme du code de sortie de l'intégrateur 8.

Le convertisseur numérique-analogique 10 permet d'appliquer à l'entrée de l'amplificateur 7, une image analogique de l'impédance statique déterminée par l'intégrateur 8.

L'intégrateur 8 du type inverseur, supprime du signal d'entrée la composante basse fréquence ($F \leq 0,05$ Hz) et présente, en fonction de l'amplification du signal, des constantes de temps de plus en plus rapides afin de permettre de suivre au plus près le signal. Ceci est particulièrement nécessaire en cas de variation importante de l'impédance statique.

Ainsi, chaque demande d'injection du microprocesseur provoque un cycle complet de mesure avec injection permettant l'asservissement.

En dehors d'une injection, la tension mesurée entre l'électrode de recueil et le boîtier correspond au bruit recueilli sur l'électrode.

Ce bruit fausse la mesure effectuée lors d'une injection et perturbe l'asservissement du stimulateur sur la ventilation-minute dans cet exemple.

Selon l'invention, la mesure du bruit est effectuée en utilisant la chaîne électronique de mesure de l'impédance (Figure 1) dans les conditions de mesure de l'impédance pulmonaire, mais sans injection de courant.

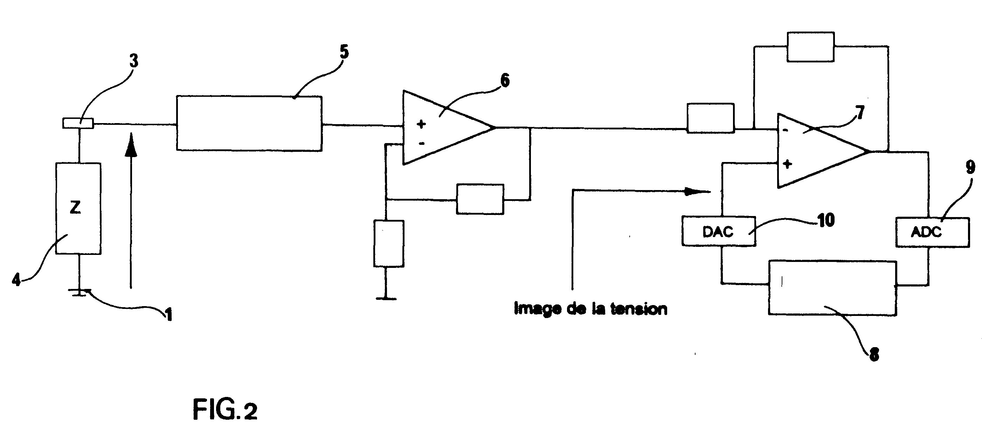

Sur la figure 2, les mêmes références désignent les mêmes éléments que sur la figure 1.

Le signal traité par la chaîne est le signal présent sur l'électrode de recueil durant un échantillonnage sans injection. Un cycle de mesure du bruit comporte les mêmes opérations qu'un cycle de mesure de l'impédance pulmonaire à l'exception de l'injection de courant. Il comporte notamment la détermination d'une impédance statique due au bruit et d'une impédance dynamique due au bruit.

A chaque cycle de mesure, la tension numérisée correspondant à l'impédance dynamique due au bruit est comparée à un seuil, de préférence programmable.

Le seuil peut être positif ou négatif. Il peut y avoir un seuil positif et un seuil négatif.

Chaque dépassement du seuil provoque par exemple l'incrémentation d'un compteur qui est lu et remis à zéro périodiquement par le microprocesseur.

Lorsque le compteur atteint une valeur prédéterminée, le microprocesseur décide la suspension de l'asservissement.

Selon un mode de réalisation de l'invention, la mesure du bruit peut être interrompue temporairement lorsque le capteur du paramètre physiologique détecte un niveau d'activité du patient correspondant à une période de repos. Par exemple, lorsque le patient est endormi, le niveau d'activité diminue et reste faible jusqu'au réveil. Pendant cette période, il n'est pas indispensable de mesurer le niveau de bruit et en conséquence, la mesure du bruit peut être interrompue. Il en résulte une économie sensible pour la consommation de courant de la pile. Lorsque le niveau d'activité du patient augmente, la mesure du bruit peut reprendre.

La reprise de la mesure du bruit peut être déclenchée par le franchissement d'un seuil prédéterminé du niveau d'activité détecté par le capteur. Ce seuil est de préférence défini par un pourcentage au dessus de la valeur minimale instantanée du paramètre mesuré.

Dans le cas où le paramètre physiologique est la ventilation-minute VE, ce seuil peut être avantageusement lié à la valeur basse VEbas telle qu'elle est définie dans le document EP-0 493 222.

Par rapport à la valeur VEbas ainsi définie, le seuil prédéterminé du niveau d'activité détecté par le capteur est défini par un écart en pourcentage de 12% par exemple, c'est-à-dire que

$$VEseuil = VEbas (100\% + 12\%) = 1,12 \ VEbas$$

Le paramètre VE est mesuré sur 4 ou 8 cycles respiratoires par exemple et sa valeur moyenne VEmoy est retenue et comparée à la valeur VEbas et à la valeur de seuil VEseuil.

Si la valeur moyenne VEmoy est inférieure à VEbas, le patient est au repos et la mesure du bruit est suspendue.

Si la valeur moyenne VEmoy est supérieure à VEseuil, le patient est en activité et la mesure du bruit est effectuée.

Pour éviter que la mesure du bruit soit suspendue et reprise trop fréquemment, une certaine hystérésis est prévue :

- lorsque la valeur moyenne VEmoy augmente et dépasse la valeur VEbas, le système reste dans son état antérieur (sans mesure du bruit) jusqu'à ce que la valeur moyenne VEmoy dépasse la valeur de seuil VEseuil, où la mesure du bruit est reprise,
- lorsque la valeur moyenne VEmoy diminue et tombe en dessous de la valeur de seuil VEseuil, le système reste dans son état antérieur (avec mesure du bruit) jusqu'à ce que la valeur moyenne VEmoy tombe en dessous de la valeur VEbas, où la mesure du bruit est suspendue.

Parmi les avantages proposés par l'invention, il faut noter les suivants :

- Tout d'abord, les mêmes éléments analogiques peuvent être utilisés pour réaliser d'abord toutes les étapes de la mesure d'asservissement, et puis celles de la mesure de bruit de manière décalée dans le temps.

- La réutilisation de la chaîne d'asservissement pour la réalisation de la mesure de bruit permet la mise en oeuvre de la détection de bruit sans intégration de nouveaux composants .

- La réalisation de la mesure de bruit lors d'un échantillonnage du même type que l'échantillonnage durant l'injection permet de détecter la présence de bruits parasites de faible valeur dus aux interférences électromagnétiques.

- La fréquence des cycles de mesure du bruit peut être modifiée. Elle peut être réalisée à chaque cycle d'injection ou à une autre fréquence.

De même, le temps entre l'injection et l'échantillonnage de la mesure de bruit peut être constant ou varier. Si la mesure de bruit a lieu à chaque cycle d'injection et avec un retard fixe par rapport à l'injection, les battements dus à un signal parasite périodique seront vus de la même façon lors de la mesure d'impédance pulmonaire et lors de la mesure du bruit. Le bruit parasite perturbant la mesure d'impédance sera donc bien détecté.

En outre, la mesure du bruit peut être suspendue temporairement, par exemple pendant les périodes de repos du patient, pour économiser la pile.

- Le fait de rendre programmables les seuils de comparaison de la tension recueillie lors de la mesure de bruit rend le système souple et le critère de détection facilement modifiable.

De même, la lecture du contenu du compteur par le microprocesseur peut être réalisée à une fréquence modifiable.

La possibilité de faire varier des paramètres comme le seuil de comparaison, la fréquence de mesure, la fréquence de lecture du nombre de dépassements, permet d'adapter le système de mesure aux bruits perturbateurs.

Le principe de détection du bruit dû aux interférences électromagnétiques peut être généralisé, la réalisation décrite ci-dessus n'en représentant qu'une des possibilités.

La tension obtenue en sortie de chaîne de recueil lors de la mesure du bruit peut être traitée (par exemple par moyenne) avant la comparaison au seuil.

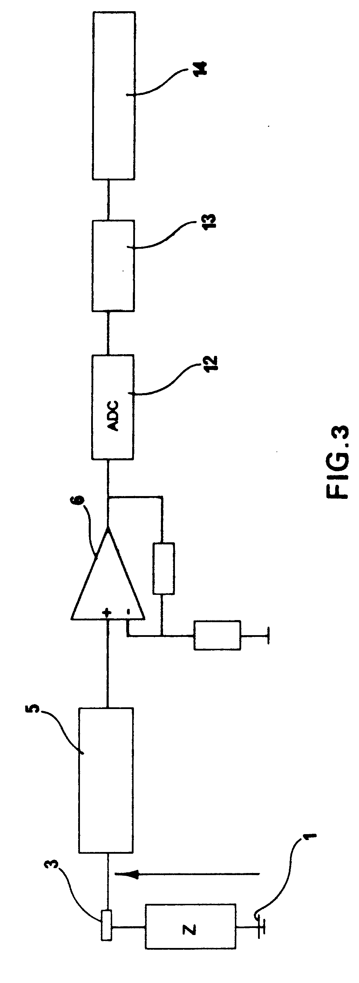

La chaîne de traitement de la tension recueillie peut être du type représenté sur la figure 3 : la tension amplifiée en 6 est numérisée en 12, filtrée en 13 et le code correspondant est lu par le microprocesseur 14.

L'intervention du microprocesseur peut être modifiée. Les seuils de détection du bruit peuvent être câblés ou programmables. Le traitement de la tension obtenue en sortie de chaîne et la suspension de l'asservissement en cas de perturbation peuvent être réalisés par le microprocesseur, par le matériel, ou par une combinaison des deux.

L'exemple décrit se rattache à un stimulateur asservi à la ventilation-minute. Il est clair que sans sortir du cadre de la présente invention, son principe peut s'appliquer à d'autres types d'asservissements où la grandeur représentative du paramètre physiologique est susceptible d'être perturbée par des interférences électromagnétiques. C'est le cas, par exemple, de l'asservissement à des paramètres tels que le volume d'éjection, la température, la concentration d'un gaz dans le sang, bien connus de l'art antérieur. Par ailleurs, la grandeur représentative du paramètre physiologique peut remplir d'autres fonctions que l'asservissement d'une fréquence de stimulation, comme par exemple la confirmation de la présence d'un trouble du rythme dans les défibrillateurs implantables.

## Revendications

1. Dispositif implantable actif comportant une chaîne électronique (5-10) de mesure d'une grandeur représentative d'un paramètre physiologique et un dispositif (14) pour assurer une fonction du dispositif implantable actif, **caractérisé en ce que** :

   - ladite grandeur est mesurée pour déterminer une valeur du bruit ;
   - ladite valeur du bruit est comparée à un seuil, et
   - lorsque la valeur du bruit dépasse le seuil, ce dépassement est pris en compte par ledit dispositif (14) en vue d'une suspension éventuelle de la fonction.

**2.** Dispositif selon la revendication 1, **caractérisé en ce que** la fonction du dispositif implantable est l'asservissement de la fréquence de stimulation cardiaque.

**3.** Dispositif selon la revendication 1, **caractérisé en ce que** le paramètre physiologique est la ventilation-minute et la grandeur représentative est la tension entre une électrode de recueil (3) et le boîtier d'un stimulateur (1).

**4.** Dispositif selon la revendication 3, **caractérisé en ce que** :

- ladite tension est mesurée pendant une injection de courant pour déterminer l'impédance pulmonaire dynamique,
- ladite tension est mesurée en l'absence d'injection de courant pour déterminer le bruit,
- la tension correspondant au bruit est comparée à une valeur de seuil, et
- lorsque la tension correspondant au bruit dépasse la valeur de seuil, ce dépassement est pris en compte par ledit dispositif (14) en vue d'une suspension éventuelle de la fonction.

**5.** Dispositif selon la revendication 3, **caractérisé en ce que** la tension est mesurée pendant une injection de courant pour déterminer l'impédance pulmonaire dynamique et en dehors d'une injection de courant pour déterminer le bruit, au moyen de la même chaîne électronique de mesure (5-10).

**6.** Dispositif selon la revendication 5, **caractérisé en ce que** l'impédance pulmonaire dynamique est définie par différence entre l'impédance pulmonaire mesurée et l'impédance pulmonaire statique obtenue par intégration des mesures précédentes.

**7.** Dispositif selon la revendication 4, **caractérisé en ce qu'**en cas de dépassement de la valeur de seuil un compteur est incrémenté et le dispositif (14) suspend la fonction lorsque le compteur atteint un total prédéterminé.

**8.** Dispositif selon la revendication 1, **caractérisé en ce que** la mesure de ladite grandeur et la détermination de la valeur du bruit sont effectuées seulement lorsque la mesure de la grandeur représentative d'un paramètre physiologique dépasse un seuil prédéterminé.

**Claims**

**1.** An active implantable device comprising an electronic chain (5-10) for measuring a representative variable of a physiological parameter and a device (14) for effecting a function of the active implantable device, **characterised in that**:

- said variable is measured to determine a noise value;
- said noise value is compared with a threshold, and
- when the noise value exceeds the threshold, this exceeding is taken into account by said device (14) with a view to possible suspension of the function.

**2.** A device according to Claim 1, **characterised in that** the function of the implantable device is the automatic control of the cardiac pacing rate.

**3.** A device according to Claim 1, **characterised in that** the physiological parameter is the minute ventilation and the representative variable is the voltage between a collecting electrode (3) and the casing of a pacemaker (1).

**4.** A device according to Claim 3, **characterised in that**:

- said voltage is measured during an injection of current to determine the dynamic pulmonary impedance,
- said voltage is measured in the absence of injection of current to determine the noise,
- the voltage corresponding to the noise is compared with a threshold value, and
- when the voltage corresponding to the noise exceeds the threshold value, this exceeding is taken into account by said device (14) with a view to possible suspension of the function.

**5.** A device according to Claim 3, **characterised in that** the voltage is measured during an injection of current to determine the dynamic pulmonary impedance and outside an injection of current to determine the noise, by means of the same electronic measurement chain (5-10).

**6.** A device according to Claim 5, **characterised in that** the dynamic pulmonary impedance is defined by the difference between the measured pulmonary impedance and the static pulmonary impedance obtained by integration of the previous measurements.

**7.** A device according to Claim 4, **characterised in that** that in the event of the threshold value being exceeded a counter is incremented and the device (14) suspends the function when the counter reaches a predetermined total.

**8.** A device according to Claim 1, **characterised in**

**that** the measurement of said variable and the determination of the noise value are effected only when the measurement of the variable representative of a physiological parameter exceeds a predetermined threshold.

## Patentansprüche

1. Aktive implantierbare Vorrichtung mit einer elektronischen Messkette (5-10) für eine Größe, die repräsentativ für einen physiologischen Parameter ist, und einer Einrichtung (14) zur Sicherstellung einer Funktion der aktiven implantierbaren Vorrichtung, **dadurch gekennzeichnet, dass**:

   - die Größe gemessen wird, um einen Geräuschwert zu bestimmen;
   - der Geräuschwert mit einer Schwelle verglichen wird und,
   - wenn der Geräuschwert die Schwelle überschreitet, dieses Überschreiten von der Einrichtung (14) im Hinblick auf eine eventuelle Unterbrechung der Funktion berücksichtigt wird.

2. Vorrichtung nach Anspruch 1, **dadurch gekennzeichnet, dass** die Funktion der implantierbaren Vorrichtung die Steuerung der Frequenz der Herzstimulation ist.

3. Vorrichtung nach Anspruch 1, **dadurch gekennzeichnet, dass** der physiologische Parameter die Minuten-Ventilation und die repräsentative Größe die Spannung zwischen einer Empfangselektrode (3) und dem Gehäuse eines Schrittmachers (1) ist.

4. Vorrichtung nach Anspruch 3, **dadurch gekennzeichnet, dass**

   - die Spannung während einer Strominjektion gemessen wird, um die dynamische Lungenimpedanz zu bestimmen,
   - die Spannung bei Abwesenheit einer Strominjektion gemessen wird, um das Geräusch zu bestimmen,
   - die dem Geräusch entsprechende Spannung mit einem Schwellenwert verglichen wird und,
   - wenn die dem Geräusch entsprechende Spannung den Schwellenwert überschreitet, dieses Überschreiten durch die Einrichtung (14) im Hinblick auf eine eventuelle Unterbrechung der Funktion berücksichtigt wird.

5. Vorrichtung nach Anspruch 3, **dadurch gekennzeichnet, dass** mittels derselben elektronischen Messkette (5-10) die Spannung während einer Strominjektion gemessen wird, um die dynamische Lungenimpedanz zu bestimmen, und außerhalb einer Strominjektion gemessen wird, um das Geräusch zu bestimmen.

6. Vorrichtung nach Anspruch 5, **dadurch gekennzeichnet, dass** die dynamische Lungenimpedanz durch die Differenz zwischen der gemessenen Lungenimpedanz und der durch die Integration der vorangehenden Messwerte erhaltenen statischen Lungenimpedanz definiert ist.

7. Vorrichtung nach Anspruch 4, **dadurch gekennzeichnet, dass** im Falle eines Überschreitens des Schwellenwerts ein Zähler inkrementiert wird und die Einrichtung (14) die Funktion unterbricht, wenn der Zähler eine vorbestimmte Gesamtgröße erreicht.

8. Vorrichtung nach Anspruch 1, **dadurch gekennzeichnet, dass** die Messung der Größe und die Bestimmung des Geräuschwerts nur vorgenommen werden, wenn der Messwert der für einen physiologischen Parameter repräsentativen Größe eine vorbestimmte Schwelle überschreitet.

FIG.2

Image de la tension

EP 0 663 219 B1

FIG.3

EP 0 663 219 B1